(19) Europäisches Patentamt
European Patent Office
Office européen des brevets

(11) Publication number: **0 136 143**
**B1**

(12) **EUROPEAN PATENT SPECIFICATION**

(45) Date of publication of patent specification: **04.11.87**

(51) Int. Cl.$^4$: **C 07 D 209/96,** A 61 K 31/40

(21) Application number: **84306332.2**

(22) Date of filing: **17.09.84**

# (54) Spiro-succinimides for treatment of diabetes complications.

(30) Priority: **23.09.83 US 535444**

(43) Date of publication of application:
**03.04.85 Bulletin 85/14**

(45) Publication of the grant of the patent:
**04.11.87 Bulletin 87/45**

(84) Designated Contracting States:
**AT BE CH DE FR GB IT LI LU NL SE**

(56) References cited:
**EP-A-0 065 392**
**US-A-3 985 888**
**US-A-4 307 108**

(73) Proprietor: **PFIZER INC.**
**235 East 42nd Street**
**New York, N.Y. 10017 (US)**

(72) Inventor: **Lipinski, Christopher Andrew**
**10 Connshire Drive**
**Waterford, CT (US)**

(74) Representative: **Moore, James William, Dr.**
**Pfizer Limited Ramsgate Road**
**Sandwich Kent CT13 9NJ (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 136 143 B1

**Description**

This invention relates to novel spiro-succinimides useful in the treatment of certain chronic complications arising from diabetes mellitus, such as diabetic cataracts, retinopathy and neuropathy, to pharmaceutical compositions containing such compounds and to a method of using these compounds.

In the past various attempts have been made to obtain more effective oral anti-diabetic agents. Generally these efforts have involved synthesis of new organic compounds, particularly sulfonyl ureas, and determination of their ability to substantially lower blood sugar levels when administered orally. However, little is known about the effect of organic compounds in preventing or alleviating chronic complications of diabetes, such as diabetic cataracts, neuropathy and retinopathy. U.S. Patent No. 3,821,383 discloses aldose reductase inhibitors like 1,3-dioxo-1H-benz[d,e]isoquinoline-2(3H)-acetic acid and derivatives thereof to be useful for the treatment of these conditions. U.S. Patent 4,117,230 teaches the use of certain hydantoins for treating complications of diabetes as aldose reductase inhibitors. Such aldose reductase inhibitors function by inhibiting the activity of the enzyme aldose reductase, which is primarily responsible for regulating the reduction of aldoses, such as glucose and galactose, to the corresponding polyols, such as sorbitol and galactitol, in humans and other animals. In this way unwanted accumulations of galactitol in the lens of galactosemic subjects and of sorbitol in the lens, of peripheral nervous cord and kidneys of various diabetic subjects are prevented or reduced. Accordingly, such compounds are of therapeutic value as aldose reductase inhibitors for controlling certain chronic diabetic complications, including those of an ocular nature, since it is known in the art that the presence of polyols in the lens of the eye leads to cataract formation, with a concomitant loss of lens clarity.

Carr et al., U.S. Patent 3,985,888, teaches certain spiroalkanone-imides and their use as sedatives. European Patent Application Publication No. 0065392 discloses certain spiro-succinimide derivatives and their use as aldose reductase inhibitors.

The compounds of the present invention are spirosuccinimides of the formula

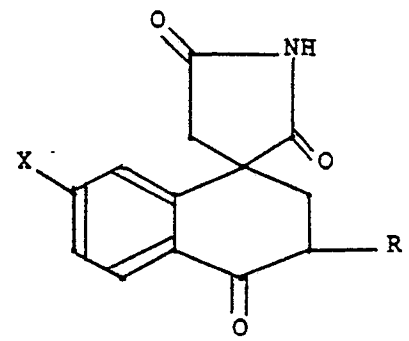

- - I

or a pharmaceutically acceptable salt thereof, wherein R is methyl or ethyl and X is fluoro or chloro.

A preferred compound is one wherein R is methyl and X is fluoro.

Both mixtures of optically active isomers and partially or completely optically resolved isomers of the compounds claimed herein are within the scope of the present invention. In particular the present invention includes a compound of the formula

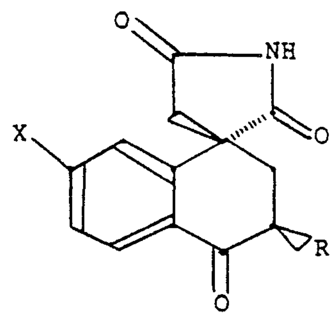

- - II

or a pharmaceutically acceptable salt thereof, wherein R and X are as defined for compounds of formula I.

A preferred compound is one wherein R is methyl and X is fluoro. Also embraced by the present invention are pharmaceutical compositions comprising a pharmaceutically acceptable carrier or diluent and a compound of formula I or II.

The numbering system of the spiro compounds of formula I or II is as shown.

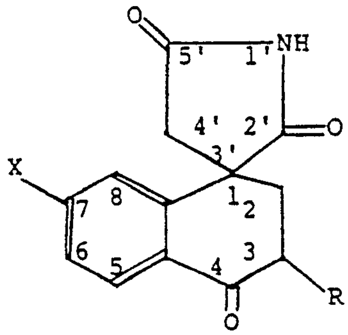

These compounds are 7-X-3-R-2,3-dihydro-spiro[naphthalene-1(4H),3'-pyrrolidine]-2',4,5'-triones.

Compounds of formula I or II can be prepared by the general method disclosed by Bastian et al., U.S. Patent 3,901,916.

According to the Bastian et al. procedure, a benzaldehyde III is reacted with dimethyl malonate IV to form a condensation product V which is then reacted with an aqueous alkali metal cyanide solution to obtain the phenyl cyano propionate VI.

The propionate VI is reacted with an acrylate of the formula $CH_2=C(R)CO_2R'$ wherein R' is lower alkyl preferably having 1—4 carbon atoms, e.g., methyl or *tert*-butyl methacrylate (R is methyl) or methyl or *tert*-butyl ethacrylate (R is ethyl), in a polar aprotic solvent such as dimethylformamide in the presence of an alkali metal alcoholate such as potassium *t*-butoxide at a temperature of between about 25 and 120°C., preferably about 65°C.

The resulting adduct VII is cyclyzed by reacting with a strong acid such as sulfuric acid, hydrochloric acid and the like, preferably concentrated or aqueous sulfuric acid, at a temperature range of between about 25 and 130°C. preferably about 70—80°C., to obtain the desired compound of formula I.

The resulting diastereomers can be separated by methods known in the art such as recrystallization with a suitable solvent such as isopropanol or trituration, for example, with an alcohol-ether solvent such as isopropanol-diethyl ether, to obtain compounds of formula II.

The term "Rel" and "(±)" each means a 1:1 racemic mixture of the two optically active enantiomers.

SYNTHETIC SCHEME

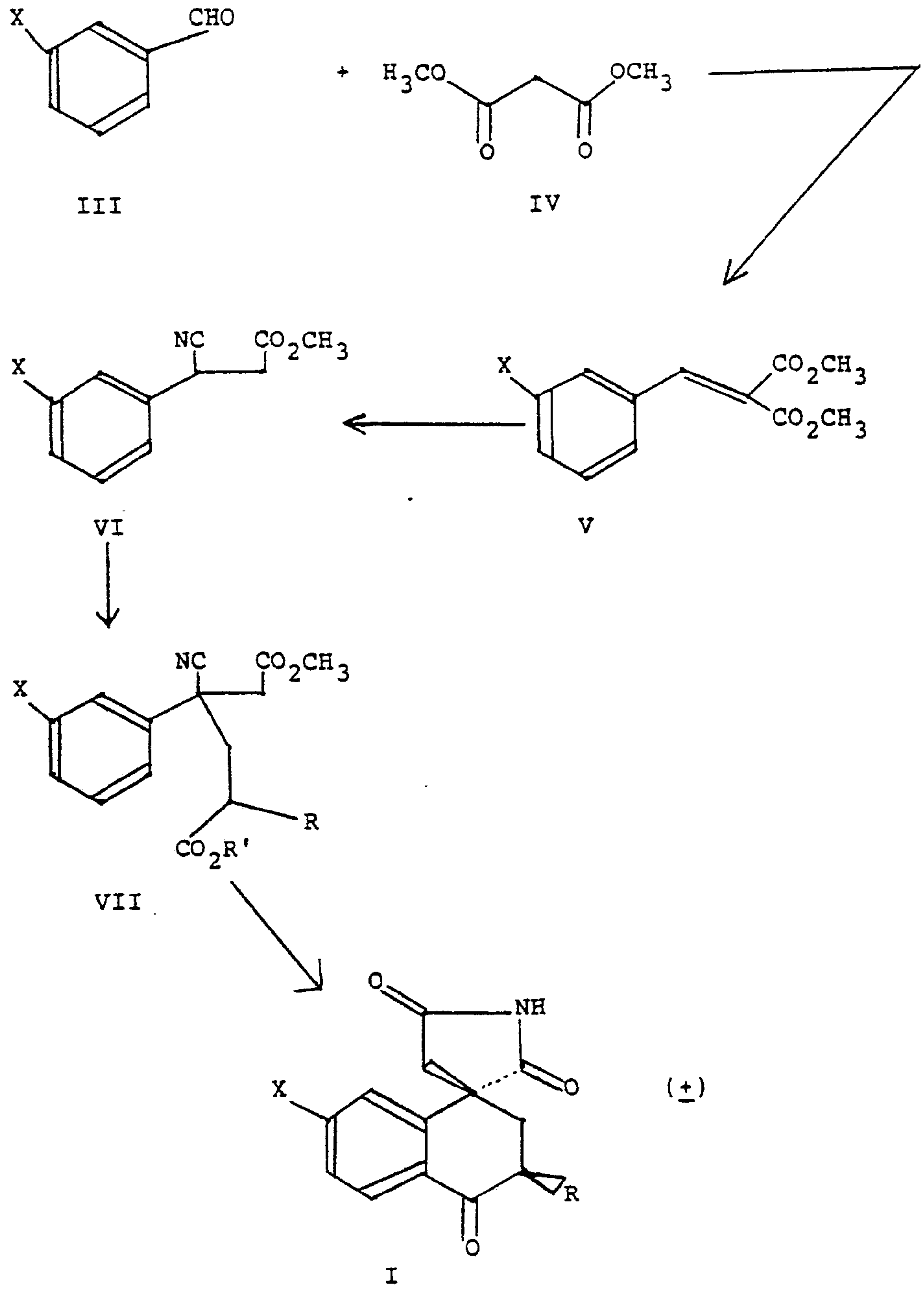

III IV

VI V

VII

I

Because of the acidic hydrogen atom in the spiro heterocyclic ring of the compounds of formula I or II, salts may be formed with pharmaceutically acceptable cations by conventional methods. Thus, these salts may be readily prepared by treating the compounds of formula I or II with an aqueous solution of the desired pharmaceutically acceptable cation and evaporating the resulting solution to dryness, preferably under reduced pressure. Alternatively, a lower alkyl alcohol solution of the compound of formula I may be mixed with an alkoxide of the desired cation and subsequently evaporating the solution to dryness. Suitable pharamaceutically acceptable cations for this purpose include, but are not limited to, alkali earth metal cations such as potassium and sodium, ammonium or water soluble amine addition salts such as the lower alkanolammonium and other base salts with organic amines which are pharmaceutically acceptable and alkaline earth metal cations such as calcium and magnesium.

Pharmaceutically acceptable salts are those which do not cause unacceptable adverse reactions when administered.

The novel compounds of formula I or II and the pharmaceutically acceptable salts thereof are useful as

inhibitors of the enzyme aldose reductase in the treatment of chronic complications of diabetes, such as diabetic cataracts, retinopathy and neuropathy. As used in the claims and specification hereof, treatment is meant to include both the prevention and alleviation of such conditions. The compound may be administered to a subject in need of treatment by a variety of conventional routes of administration, including orally, parenterally and topically. In general, these compounds will be administered orally or parenterally at dosages between about 0.05 and 25mg./kg. body weight of the subject to be treated per day, preferably from about 0.1 to 10 mg./kg. per day. However, some variation in dosage will necessarily occur depending on the condition of the subject being treated. The person responsible for administration will, in any event, determine the appropriate dose for the individual subject.

The novel compound of the invention may be administered alone or in combination with pharmaceutically acceptable carriers, in either single or multiple doses. Suitable pharmaceutical carriers include inert solid diluents or fillers, sterile aqueous solution and various organic solvents. The pharmaceutical compositions formed by combining the novel compounds of formula I and the pharmaceutically acceptable carriers are then readily administered in a variety of dosage forms such as tablets, powders, lozenges, syrups, injectable solutions and the like. These pharmaceutical compositions can, if desired, contain additional ingredients such as flavorings, binders, excipients and the like. Thus, for purposes of oral administration, tablets containing various excipients such as sodium citrate, calcium carbonate and calcium phosphate may be employed along with various disintegrants such as starch, alginic acid and certain complex silicates, together with binding agents such as polyvinylpyrrolidone, sucrose, gelatin and acacia. Additionally, lubricating agents such as magnesium stearate, sodium lauryl sulfate and talc are often useful for tabletting purposes. Solid compositions of a similar type may also be employed as fillers in soft and hard filled gelatin capsules. Preferred materials for this include lactose or milk sugar and high molecular weight polyethylene glycols. When aqueous suspensions or elixirs are desired for oral administration, the essential active ingredient therein may be combined with various sweetening or flavoring agents, coloring matter or dyes and, if desired, emulsifying or suspending agents, together with diluents such as water, ethanol, propylene glycol, glycerin and combinations thereof.

For parenteral administration, solutions of the novel compound of formula I or II in sesame or peanut oil, aqueous propylene glycol, or in sterile aqueous solutions may be employed. Such aqueous solutions should be suitably buffered if necessary and the liquid diluent first rendered isotonic with sufficient saline or glucose. These particular aqueous solutions are especially suitable for intravenous, intramuscular, subcutaneous and intraperitoneal administration. In this connection, the sterile aqueous media employed are all readily available by standard techniques known to those skilled in the art.

Compounds of formula I or II may not only be advantageously employed for the preparation of aqueous pharmaceutical compositions for parenteral administration, as described above, but more particularly for the preparation of pharmaceutical compositions suitable for use as ophthalmic solutions. Such ophthalmic solutions are of principal interest for the treatment of diabetic cataracts by topical administration and the treatment of such conditions in this manner is a preferred embodiment of the present invention. Thus, for the treatment of diabetic cataracts the compounds of this invention are administered to the eye of the subject in need of treatment in the form of an ophthalmic preparation prepared in accordance with conventional pharmaceutical practice, see for example "Remington's Pharmaceutical Sciences" 15th Edition, pages 1488 to 1501 (Mack Publishing Co., Easton, Pa). The ophthalmic preparation will contain a compound of formula I, II or a pharmaceutically acceptable salt therof in a concentration from about 0.01 to about 1% by weight, preferably from about 0.05 to about 0.5% in a pharmaceutically acceptable solution, suspension or ointment. Some variation in concentration will necessarily occur, depending on the particular compound employed, the condition of the subject to be treated and the like, and the person responsible for treatment will determine the most suitable concentration for the individual subject. The ophthalmic preparation will preferably be in the form of a sterile aqueous solution containing, if desired, additional ingredients, for example preservatives, buffers, tonicity agents, antioxidants and stabilizers, nonionic wetting or clarifying agents, viscosity-increasing agents and the like. Suitable preservatives include benzalkonium chloride, benzethonium chloride, chlorobutanol, thimerosal and the like. Suitable buffers include boric acid, sodium and potassium bicarbonate, sodium and potassium borate, sodium and potassium carbonate, sodium acetate, sodium biphosphate and the like, in amounts sufficient to maintain the pH at between about 6 and 8, preferably between about 7 and 7.5. Suitable tonicity agents are dextran 40, dextran 70, dextrose, glycerin, potassium chloride, propylene glycol, sodium chloride, and the like, such that the sodium chloride equivalent of the ophthalmic solution is in the range 0.9 plus or minus 0.2%. Suitable antioxidants and stabilizers include sodium bisulfite, sodium metabisulfite, sodium thiosulfite, thiourea and the like. Suitable wetting and clarifying agents include polysorbate 80, polysorbate 20, poloxamer 282 and tyloxapol. Suitable viscosity-increasing agents include dextran 40, dextran 70, gelatin, glycerin, hydroxyethylcellulose, hydroxmethyl-propylcellulose, lanolin, methylcellulose, petrolatum, polyethylene glycol, polyvinyl alcohol, polyvinyl-pyrrolidine, carboxymethylcellulose and the like. The ophthalmic preparation will be administered topically to th eye of the subject in need of treatment by conventional methods, for example in the form of drops or by bathing the eye in the ophthalmic solution.

The activity of the compounds of the present invention as agents for the control of chronic diabetic complications may be determined by a number of standard biological or pharmacological tests. Suitable

tests include (1) measuring their ability to inhibit the enzyme activity of isolated aldose reductase; (2) measuring their ability to reduce or inhibit sorbitol accumulation in the sciatic nerve and lens of acutely streptozotocinized, i.e. diabetic, rats; (3) measuring their ability to reverse already-elevated sorbitol levels in the sciatic nerve and lens of chronic streptozotocin-induced diabetic rats; (4) measuring their ability to prevent or inhibit galacticol formation in the lens of acutely galactosemic rats; (5) measuring their ability to delay cataract formation and reduce the severity of lens opacities in chronic galactosemic rats; (6) measuring their ability to prevent sorbitol accumulation and cataract formation in isolated rat lens incubated with glucose; and (7) measuring their ability to reduce already elevated sorbitol levels in isolated rat lens incubated with glucose.

The present invention is illustrated by the following examples. It will be understood, however, that the invention is not limited to the specific details of these examples. Proton nuclear magnetic resonance spectra (NMR) were measured at 250 MHz (unless otherwise indicated) for solutions in perdeuterodimethyl sulfoxide (DMSO-$d_6$) and peak positions are expressed in parts per million (ppm) downfield from tetramethylsilane. The peak shapes are denoted as follows: s, singlet, d, doublet, t, triplet; q, quartet; m, multiplet; b, broad.

## Example

Rel 1,3'S,3R Spiro[naphthalene-1(4H),3'-pyrrolidine]-2',4,5'-trione, 2,3-dihydro-7-fluoro-3-methyl

A. 12.5 g. of crude 5-carboxy-3-cyano-3-(3-fluorophenyl)-5-methyl-hexanoic acid dimethyl ester was combined with 62.5 ml. concentrated sulfuric acid and was heated at 70°C. for 2.5 hours. The reaction was quenched over ice and extracted with three 100 ml. portions of ethyl acetate. The ethyl acetate was extracted with pH 11 aqueous sodium hydroxide. The aqueous base layer was washed with ethyl acetate and the aqueous layer was adjusted to pH 2 with hydrochloric acid and extracted with four 100 ml. portions ethyl acetate. After drying over anhydrous sodium sulfate the ethyl acetate was concentrated in vacuo to an oil and eventually a white foam. This was triturated for 20 hours with isopropyl alcohol-diethyl ether to give after drying at 110°C. at reduced pressure 610 mg. of the title compound: m.p.: shrinks at 200—202°C., decomposes at 212—215°C. Anal. ($C_{14}G_{12}O_3NF$).

Calcd: C, 64.36; H, 4.63; N, 5.36. Found: c, 64.25; H, 4.60; N, 5.42. IR(KBr): 3228, 1723, 1705, 1692, 1605, 1343, 1236, 1199, 1186 $cm^{-1}$. NMR 250 MHZ ($CD_3SOCD_3$): 11.7(very br s, 1;, 7.97(m, 1), 7.31(m, 1), 7.13(m, 1), 3.5—2.1(m, 5), and 1.16(d, 3) ppm. Peaks attributable to a minor diastereomer (major/minor ratio = 9) were observed at 7.47 ppm (aromatic H) and 1.12 ppm (one of two peaks of a $CH_3$ doublet). Decoupling of the methyl group protons sharpens the resonance of the keto carbonyl signal but does not affect either of the imido carbonyl groups.

This experiment defines the structure of the major diastereomer as the title spiro[naphthalene pyrrolidine]trione rather than the alternate indanone spiro methyl glutarimide cyclization product. Titration of the product with 0.5N sodium hydroxide gave a pkA value of 9.59 in 1:1 dioxane-water and 8.25 in water, values which are consistent with a succinimide but not a glutarimide.

B. The procedure of A was employed using a aqueous solution containing 90% sulfuric acid instead of concentrated sulfuric acid and heating to 80°C. for 1—2 hours instead of 70°C for 2.5 hours. The desired reaction product was isolated in 15—18 percent crude yield and recrystallized from isopropanol to obtain 92.8 diastereomic mixture with the same major diastereomer as in A.

## Preparation

5-Carboxy-3-cyano-3-(3-fluorophenyl)-5-methyl-hexanoic acid dimethyl ester

18.66 g (0.09 mol) of 3-cyano-3-(3-fluorophenyl)propionic acid methyl ester (prepared according to the general procedure described in U.S. Patent 3,901,916 — column 10) was combined with 9.33 g. (0.006 mol) of potassium *tert*-butoxide and 90 ml. of dry dimethylformamide were combined and heated at 65°C for 21 hours. The reaction was poured onto dilute hydrochloric acid solutons and then was extracted with three 300 ml. portions of ethyl acetate. The ethyl acetate was concentrated to 400 ml., washed with two 300 ml. portions water, 200 ml. brine and dried over anhydrous sodium sulfate and concentrated to give 27.76 g. of crude oil containing the title compound. NMR 60MHZ ($CDCl_3$) showed a doublet of doublets at 1.2 and 1.04 ppm, J = 8HZ corresponding to a 60:40 mixture of diastereomers.

**Claims for the Contracting States: BE CH DE FR GB IT LI LU NL SE**

1. A compound of the formula

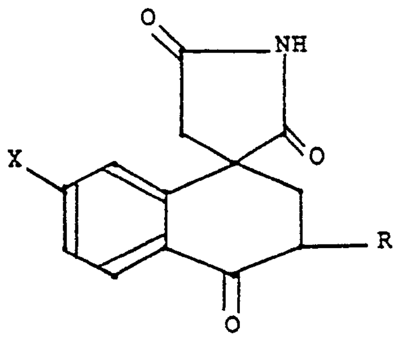

-- I

or a pharmaceutically acceptable salt thereof, wherein
R is methyl or ethyl; and
X is fluoro or chloro.

2. A compound according to claim 1 wherein R is methyl and X is fluoro.

3. A pharmaceutical composition comprising a compound according to claim 1 and a pharmaceutically acceptable carrier or diluent.

4. A pharmaceutical composition according to claim 3 wherein R is methyl and X is fluoro.

5. A compound of the formula

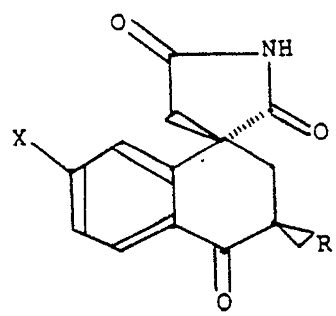

-- II

or a pharmaceutically acceptable salt thereof, wherein
R is methyl or ethyl; and
X is fluoro or chloro.

6. A compound according to claim 5 wherein R is methyl and X is fluoro.

7. A pharmaceutical composition comprising a compound according to claim 5 and a pharmaceutically acceptable carrier or diluent.

8. A pharmaceutical composition according to claim 7 wherein R is methyl and X is fluoro.

**Claims for the Contracting State: AT**

1. A process for preparing a compound of formula:

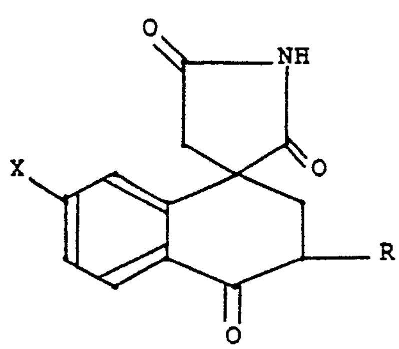

-- I

or a pharmaceutically acceptable salt thereof, wherein
R is methyl or ethyl; and

X is fluoro or chloro;
characterized by the step of cyclysing a compound of the formula:

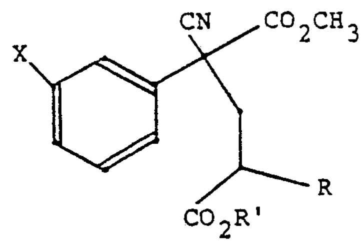

--- VII

wherein R' is lower alkyl, by reacting with a strong acid to obtain a compound of formula I.

2. A process according to claim 1 wherein R is methyl and X is fluoro.

3. A process for preparing a compound of formula:

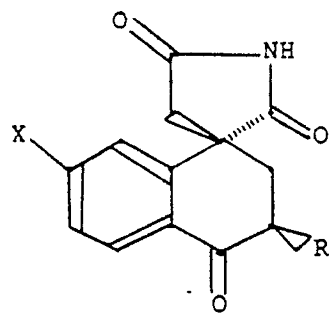

-- II

or a pharmaceutically acceptable salt thereof, whereas
R is methyl or ethyl; and
X is fluoro or chloro;
characterized by the step of cyclysing a compound of the formula:

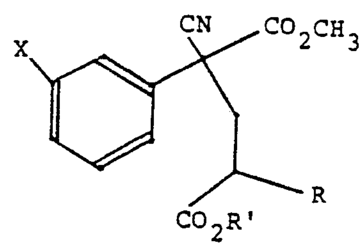

--- VII

wherein R' is lower alkyl, by reacting with a strong acid followed by separation of the mixture of diastereomers by recrystallisation or trituration to obtain a compound of formula II.

4. A process according to claim 3 wherein R is methyl and X is fluoro.

**Patentansprüche für die Vertragsstaaten: BE CH DE FR GB IT LI LU NL SE**

1. Eine Verbindung der Formel

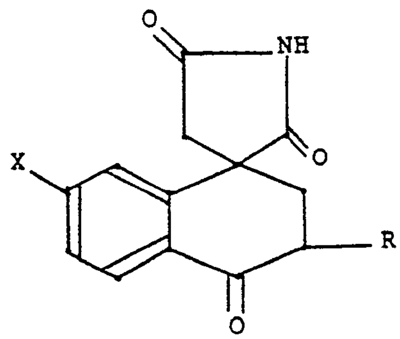

-- I

oder ein pharmazeutisch annehmbares Salz hievon, worin R Methyl oder Äthyl bedeutet und X Fluor oder Chlor ist.

2. Verbindung nach Anspruch 1, worin R Methyl und X Fluor bedeuten.

3. Pharmazeutische Zusammensetzung umfassend eine Verbindung nach Anspruch 1 und einen pharmazeutisch annhmbaren Träger oder ein Verdünnungsmittel.

4. Pharmazeutische Zusammensetzung nach Anspruch 3, worin R Methyl und X Fluor bedeuten.

5. Eine Verbindung der Formel

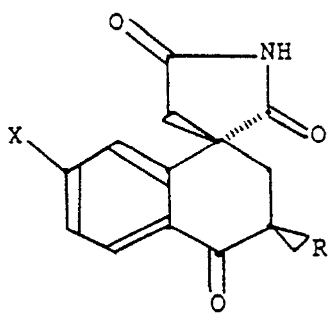

-- II

oder ein pharmazeutisch annehmbares Salz hievon, worin R Methyl oder Äthyl bedeutet und X fluor oder Chlor ist.

6. Verbindung nach Anspruch 5, worin R Methyl und X Fluor bedeuten.

7. Pharmazeutische Zusammensetzung umfassend eine Verbindung nach Anspruch 5 und einen pharmazeutisch annehmbaren Träger oder ein Verdünnungsmittel.

8. Pharmazeutische Zusammensetzung nach Anspruch 7, worin R Methyl und X Fluor bedeuten.

**Patentansprüche für den Vertraggsstaat: AT**

1. Verfahren zur Herstellung einer Verbindung der Formel

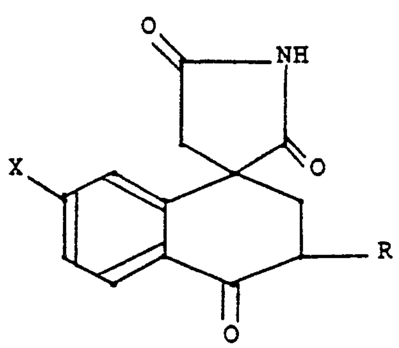

-- I

oder eines pharmazeutisch annehmbares Salz hievon, worin R Methyl oder Äthyl bedeutet und X Fluor oder Chlor ist, dadurch gekennzeichnet, daß man eine Verbindung der Formel

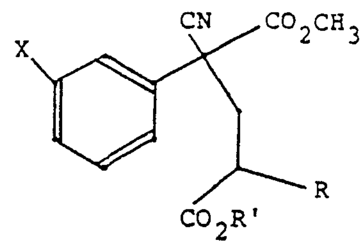

- - - VII

worin R′ nied.Alkyl ist, durch Umsetzen mit einer starken Säure cyclisiert, um eine Verbindung der Formel (I) zu erhalten.

2. Verfahren nach Anspruch 1, worin R Methyl und X Fluor bedeuten.

3. Verfahren zur Herstellung einer Verbindung der Formel

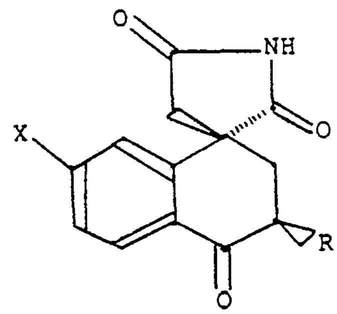

- - II

oder eines pharmazeutisch annehmbaren Salzes hievon, worin R Methyl oder Äthyl bedeutet und X Fluor oder Chlor ist, dadurch gekennzeichnet, daß man eine Verbindung der Formel

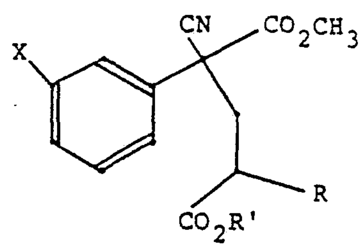

- - - VII

worin R′ nied.Alkyl ist, durch Umsetzen mit einer starken Säure cyclisiert, worauf die Diastereomerenmischung durch Umkristallisieren oder Zerreiben getrennt wird, um eine Verbindung der Formel (II) zu erhalten.

4. Verfahren nach Anspruch 3, worin R Methyl und X Fluor bedeuten.

**Revendications pour les Etats contractants: BE CH DE FR GB IT LI LU NL SE**

1. Composé de formule

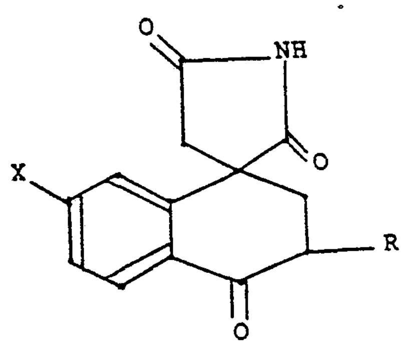

- - I

ou sel pharmaceutiquement acceptable d'un tel composé dans laquelle:

R est un groupe méthyle ou éthyle; et

X est un groupe fluoro ou chloro.

2. Composé selon la revendication 1, dans lequel R est un groupe méthyle et X est un groupe fluoro.

3. Composition pharmaceutique comprenant un composé selon la revendication 1 et un support ou diluant pharmaceutiquement acceptable.

4. Composition pharmaceutique selon la revendication 3, dans lequel R est un groupe méthyle et X est un groupe fluoro.

5. Composé de formule

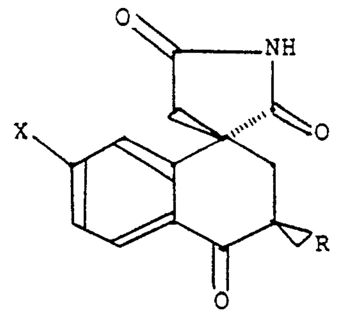

- - II

ou sel pharmaceutiquement acceptable d'un tel composé dans laquelle:

R est un groupe méthyle ou éthyle; et

X est un groupe fluoro ou chloro.

6. Composé selon la revendication 5, dans lequel R est un groupe méthyle et X est un groupe fluoro.

7. Composition pharmaceutique comprenant un composé selon la revendication 5 et un véhicule ou diluant pharmaceutiquement acceptable.

8. Composition pharmaceutique selon la revendication 7, dans lequel R est un groupe méthyle et X est un groupe fluoro.

**Revendications pour l'Etat contractant: AT**

1. Procédé de préparation d'un composé de formule

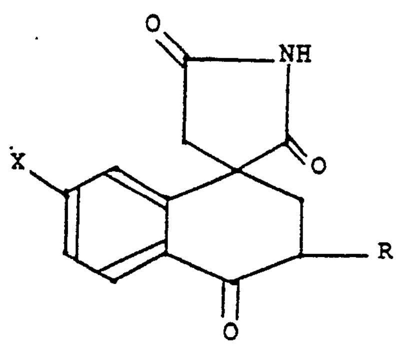

- - I

ou d'un sel pharmaceutiquement acceptable d'un tel composé, dans laquelle:

R est un groupe méthyle ou éthyle; et

X est un groupe fluoro ou chloro;

caractérisé en ce qu'il consiste à cycliser un composé de formule

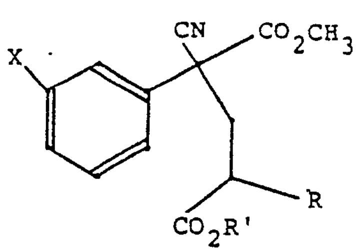

- - - VII

dans laquelle R' est un radical alkyle inférieur, par réaction avec un acide fort pour obtenir un composé de formule I.

2. Procédé selon la revendication 1, dans lequel R est un groupe méthyle et X est un groupe fluoro.

3. Procédé de préparation d'un composé de formule

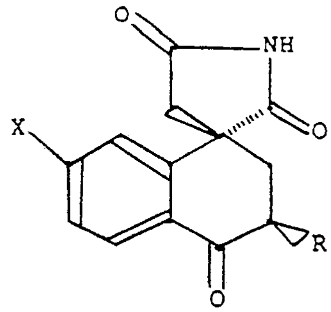

-- II

ou d'un sel pharmaceutiquement acceptable d'un tel composé, dans laquelle:
R est un groupe méthyle ou éthyle; et
X est un groupe fluoro ou chloro;
caractérisé en ce qu'il consiste à cycliser un composé de formule

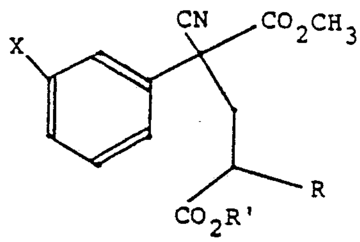

--- VII

dans laquelle R' est un groupe alkyle inférieur par réaction avec un acide fort suivi de la séparation du mélange de diastéréomères par recristallisation ou trituration pour obtenir un composé de formule (II).

4. Procédé selon la revendication 3, dans laquel R est un groupe méthyle et X est un groupe fluoro.